# EUROPÄISCHE PATENTSCHRIFT

(11) **EP 0 841 315 B1**
(45) Veröffentlichungstag und Bekanntmachung des Hinweises auf die Patenterteilung: **10.01.2001**
(21) Anmeldenummer: 97118783.6
(22) Anmeldetag: 29.10.1997
(51) Int. Cl.: C07C 2/08, C07C 7/163, C07C 5/25

(54) **Verfahren zur Oligomerisierung von i-Buten**
Process for the oligomerisation of i-butene
Procédé pour l'oligomérisation de l'i-butène

(30) Priorität: 11.11.1996 DE 19646405
(43) Veröffentlichungstag der Anmeldung: 13.05.1998
(73) Patentinhaber: EC ERDÖLCHEMIE GMBH, D-50769 Köln (DE)
(72) Erfinder: Herwig, Jens, Dr., 50859 Köln (DE); Bister, Hans-Jürgen, Dr., 41470 Neuss (DE); Stüwe, Arnd, Dr., 51373 Leverkusen (DE)
(74) Vertreter: Zobel, Manfred, Dr.

(56) Entgegenhaltungen:
- EP-A- 0 170 182
- BE-A- 735 727
- W. KRÖNIG ET AL: "Oligomerisierung von Butenen" ERDÖL UND KOHLE ERDGAS PETROCHEMIE., Bd. 19, Nr. 7, Juli 1966, LEINFELDEN DE, Seiten 497-500, XP002053199

## Beschreibung

Die vorliegende Erfindung betrifft ein Verfahren zur Oligomerisierung von i-Buten, das in einem C₄-Raffinat I enthalten ist, und umfaßt die Schritte einer ersten Hydroisomerisierung des C₄-Raffinats I, einer darauf folgenden destillativen Abtrennung von Buten-2 und n-Butan, einer zweiten Hydroisomerisierung eines Teils des Kopfprodukts der destillativen Abtrennung, deren Reaktionsprodukt in die destillative Trennung zurückgeführt wird, und schließlich der eigentlichen Oligomerisierung des i-Butens des restlichen Kopfprodukts der destillativen Abtrennung.

Die Oligomeren des i-Butens sind vorwiegend das Diisobutylen(C₈), das Triisobutylen (C₁₂), das Tetraisobutylen (C₁₆), das Pentaisobutylen(C₂₀) und weitere, deren Anteile mit höherwerdendem Oligomerisierungsgrad immer weiter abnehmen. Solche Oligomere enthalten noch je eine Doppelbindung; sie werden vorwiegend vollständig hydriert und dienen dann als absolut aromatenfreie Lösungsmittel der höheren Siedelagen in der kosmetischen und pharmazeutischen Industrie. Solche aromatenfreien Lösungsmittel sind ansonsten nur schwierig und daher kostspielig zu erhalten. Die nicht hydrierten, noch eine Doppelbindung enthaltenden Oligomeren können weiterhin der Oxosynthese oder der Veresterung zugeführt werden, wobei beispielsweise aus Diisobuten (DIB) Isononylalkohol und weiterhin Dinonylphthalat oder bei direkter Veresterung des DIB Dioctylphthalat hergestellt werden, die als Weichmacher für Thermoplaste Verwendung finden. Triisobuten kann ferner zu Dodecylmercaptan, einem Kautschukhilfsmittel, verarbeitet werden. Schließlich sei der Zusatz von dimeren Butenen oder durch Hydrierung daraus hervorgehenden Octanen zum Motorkraftstoff erwähnt.

Es ist bereits bekannt, Butene an sauren Katalysatoren, wie beispielsweise sauren Ionenaustauschern zu oligomerisieren. (Erdöl und Kohle, Erdgas, Petrochemie 19 (1966), 497-500; Hydrocarbon Processing 1973, 171-173). Nachteilig bei solchen Verfahren ist das gleichzeitige Entstehen von sogenanntem Codimer aus je einem Molekül i-Buten und Buten-1. Dieses Codimer hat weniger erwünschte Eigenschaften und stellt somit eine Verlustquelle für die Oligomeren des i-Butens dar. Eine wirksame Unterdrückung dieses Codimer wäre nur möglich, wenn es gelänge, das Buten-1 aus dem C₄-Raffinat I abzutrennen. Eine solche Abtrennung ist jedoch wegen der außerordentlich engen Siedepunktslage von i-Buten und Buten-1 unter vernünftigem Aufwand nicht möglich. Es besteht daher die Aufgabe, Buten-1 zu entfernen, um i-Buten isolieren zu können. Eine Möglichkeit dazu bietet die Isomerisierung von Buten-1 zu Buten-2 (cis und trans), welches destillativ von i-Buten abgetrennt werden kann. Wie in US 4 132 745 beschrieben, gelingt eine solche Hydroisomerisierung jedoch nur bis zu Rest -Buten-1-Gehalten von ca. 2,5 Gew.-%.

Es wurde nun gefunden, daß die weitgehende Befreiung des C₄-Raffinats von Buten-1 erreicht werden kann, so daß in der anschließenden Oligomerisierung vorteilhafte Effekte bezüglich der Ausbeute an Oligomerisierungsprodukten und bei der Verschiebung des Oligomeren-Spektrums zu höheren Oligomeren erreicht werden können.

Die Erfindung betrifft ein Verfahren zur Oligomerisierung von in einem C₄-Raffinat I enthaltenem i-Buten, das dadurch gekennzeichnet ist, daß man
a) das C₄-Raffinat I einer ersten Hydroisomerisierung an einem Edelmetallkatalysator unterwirft, wobei bei 30-80°C, 5-30 bar, einer LHSV von 5-30 h⁻¹ und einer Menge von 3-20 NL gasförmigem H₂ pro Liter C₄-Raffinat I, die über die zur Hydrierung hoch ungesättigter Komponenten im C₄-Raffinat benötigte Menge H₂ hinausgeht, gearbeitet wird,
b) das Hydroisomerisierungsprodukt von a) fraktioniert destilliert, wobei bei 5-25 bar und der sich zu diesem Druck einstellenden Temperatur gearbeitet wird, und Buten-2 und n-Butan als Sumpfablauf und i-Buten und i-Butan als Kopfprodukt abgenommen werden,
c) das Kopfprodukt von b) kondensiert, in zwei Teile teilt und den ersten Teil einer zweiten Hydroisomerisierung an einem Edelmetallkatalysator unterwirft, wobei bei 30-80°C, 5-30 bar, einer LHSV von 5-30 h⁻¹ und einer Menge von 0,3-5 NL gasförmigem H₂ pro Liter C₄-Raffinat I gearbeitet wird und die Hydroisomerisierungsbedingungen in a) und c) gleich oder verschieden sind, und das Hydroisomerisierungsprodukt von c) in das obere Drittel der Fraktionierungskolonne von b) zurückführt und
d) den zweiten Teil des kondensierten Kopfprodukts von b) bei 30-140°C an einem sauren Katalysator oligomerisiert.

Das C₄-Raffinat I wird aus dem rohen C₄-Destillatschnitt eines Crackerprodukts dadurch erhalten, daß man das darin enthaltene Butadien-1,3 durch Extraktion entnimmt und als Wertstoff weiter verwendet. Bei dieser Extraktion werden auch andere hoch ungesättigte Kohlenwasserstoffe, wie Vinylacetylen, Butadien-1,2 und andere Acetylenverbindungen aus dem rohen C₄-Destillationsschnitt entfernt. Das nach der Extraktion vorliegende C₄-Raffinat I enthält danach nur noch untergeordnete Mengen an hoch ungesättigten Verbindungen. Die zahlenmäßig überwiegenden Anteile in C₄-Raffinat I sind n-Butan, i-Butan, n-Buten-1, Buten-2 (cis und trans) und i-Buten. In unbedeutenden Anteilen können weiterhin C₃- und C₅-Kohlenwasserstoffe vorliegen.

Die erste Hydroisomerisierung als Reaktionsschritt a) des erfindungsgemäßen Verfahrens wird bei einer Temperatur von 30-80°C, bevorzugt 40-60°C und einem Druck von 5-30 bar, bevorzugt 10-20 bar, durchgeführt. Es wird eine LHSV (Liquid Hourly Space Velocity) von 5-30 h⁻¹, bevorzugt von 7-20 h⁻¹, besonders bevorzugt von 7-12 h⁻¹, eingestellt. Die Menge an zugesetztem, gasförmigem Wasserstoff muß zwei Aufgaben gerecht werden, nämlich zum einen der vollständigen Entfernung der genannten hochungesättigten Verbindungen und zum anderen der Bereitstellung einer für die Hydroisomerisierung als optimal erkannten H₂-Menge. Aus den genannten hochungesättigten Kohlenwasserstoffen entstehen hierbei unter den Hydroisomerisierungsbedingungen durch Partialhydrierung einfach ungesättigte C₄-Kohlenwasserstoffe der im C₄-Raffinat I bereits vorhandenen Art. Die H₂-Menge zur Beseitigung der hochungesättigten C₄-Kohlenwasserstoffen richtet sich selbstverständlich nach dem Umfang ihres Vorhandenseins und kann durch analytische Mittel leicht und in einer dem Fachmann bekannten Weise bestimmt werden. Die zusätzlich zu dieser H₂-Menge erforderliche weitere Menge beträgt 2-15 NL gasförmigen H₂ pro Liter C₄-Raffinat I. In bevorzugter Weise beträgt diese H₂-Menge 3-10 NL gasförmigen H₂ pro Liter flüssigen C₄-Raffinat I.

Zur Hydroisomerisierung liegt das C₄-Raffinat I in der flüssigen Phase vor. Es kann hierbei von oben nach unten in der Rieselphase über den Kontakt gefahren werden, oder der mit dem Katalysator ausgerüstete Reaktor wird von unten nach oben, also in gefluteter Form, befahren. Auch der gasförmige Wasserstoff kann im Gleichstrom oder im Gegenstrom zum C₄-Raffinat I geführt werden. Die bevorzugte Fahrweise ist die mit einem geflutetem Reaktor, wobei der Wasserstoff im Gleichstrom geführt wird.

Als Katalysatoren für die Hydroisomerisierung eignen sich alle Edelmetall-Hydrierkatalysatoren auf Trägern. Als Edelmetalle kommen hierbei in Frage: Ru, Rh, Pd, Ir und Pt, bevorzugt Ru, Pd und Pt, besonders bevorzugt Pd. Träger, auf denen solche Edelmetalle angebracht sein können, sind beispielsweise Al₂O₃ der verschiedenen Modifikationen, SiO₂, Kohle, Kieselgur und Salze, wie BaSO₄ u.a. Es hat sich als günstig erwiesen, die Edelmetalle der genannten Art durch Zusatz von Schwefelverbindungen in ihrer Aktivität zu dämpfen und damit optimal einzustellen.

Im Reaktionsschritt b) des erfindungsgemäßen Verfahrens wird das Hydroisomerisierungsprodukt von a) fraktioniert destilliert. Hierbei wird bei einem Druck von 5-25 bar und der sich unter Destillationsbedingungen zu diesem Druck einstellenden Temperatur gearbeitet. Als typische Temperaturen stellen sich bei einem Arbeitsdruck von 12 bar 25°C am Kopf der Kolonne und 88°C im Sumpf der Kolonne ein. Ein bevorzugter Druckbereich liegt bei 10-20 bar. Ein niedrigerer Druck als der angegebene Unterwert von 10 bar führt zu weiterhin erniedrigten Kopftemperaturen, die zur anschließenden Kondensation ein unökonomisch teures Kühlmedium erfordern. In weiterhin bevorzugter Weise wird in den Reaktionsschritten a), b) und c) des erfindungsgemäßen Verfahrens beim gleichen Druck gearbeitet, und es werden lediglich die erforderlichen Temperaturen angeglichen.

Als Sumpfprodukt im Reaktionsschritt b) erhält man ein Gemisch, das im wesentlichen aus n-Butan und Buten-2 besteht; Buten-2 ist hierbei das ursprünglich in C₄-Raffinat I vorliegende und das durch Hydroisomerisierung aus Buten-1 entstandene. Buten-2 ist ein geschätztes Ausgangsmaterial für die Herstellung von Alkylatbenzin, für die Oxidation zu Methyl-Ethyl-Keton und gibt schließlich bei der Rückführung in den Cracker eine erhöhte Ausbeute an Ethylen, verglichen mit anderen C₄-Kohlenwasserstoffen.

Das Kopfprodukt der Destillation gemäß Reaktionsschritt b) des erfindungsgemäßen Verfahrens besteht im wesentlichen aus i-Buten und i-Butan und nur noch untergeordneten Mengen Buten-1. Es wird kondensiert und danach in zwei Anteile geteilt. Der erste Teil wird einer zweiten Hydroisomerisierung (Reaktionsschritt c)) zugeführt, die im Rahmen der oben für die erste Hydroisomerisierung genannten Bedingungen abläuft Es ist jedoch bevorzugt, die LHSV im zweiten Hydroisomerisierungsschritt tendenziell höher einzustellen als im ersten Hydromerisierungsschritt, beispielsweise allgemein auf einen Wert von 5-30 h⁻¹, bevorzugt 10-25 h⁻¹, in Übereinstimmung mit dem ersten Hydroisomerisierungsschritt, jedoch besonders bevorzugt 15-25 h⁻¹ für den zweiten Hydroisomerisierungsschritt. Auch im zweiten Hydroisomerisierungsreaktor kann das Kondensat von oben nach unten oder von unten nach oben, bevorzugt von unten nach oben geführt werden, wobei auch hier wieder der Wasserstoff im Gleichstrom oder im Gegenstrom geführt wird. Das ablaufende zweite Hydroisomerisat wird in das obere Drittel der Fraktionierungskolonne zurückgeführt, so daß Buten-2, das im zweiten Hydroisomerisierungsreaktor gebildet wird, zusätzlich in den Sumpf der Fraktionierkolonne getrieben wird. Die Frisch-Wasserstoffdosierung kann niedriger gewählt werden als bei der 1. Isomerisierung, d.h. 0,3-5 NL H₂ pro 1 flüssiges C₄, da man im zweiten Hydroisomerisierungsreaktor nicht mehr mit hochungesättigten Verbindungen rechnen muß, so daß ein hierfür zusätzlich aufzuwendender Anteil an Wasserstoff entfallen kann.

Das Verhältnis der Teilströme des kondensierten Kopfprodukts der Fraktionierkolonne wird so eingestellt, daß 10-40 Volumteile, bevorzugt 20-30 Volumteile, als ein erster Teil der zweiten Hydroisomerisierung zugeführt wird, bezogen auf 1 Volumteil des zweiten Teils, der der Oligomerisierung zugeführt wird. Da der der Oligomerisierung zugeführte zweite Volumteil gemeinsam mit dem Sumpfablauf der Fraktionierkolonne dem gesamten Einsatz an C₄-Raffinat I in die erste Hydroisomerisierung entspricht, ist dem durch das erfindungsgemäße Verfahren durchlaufenden Stoffstrom ein Kreislauf durch den zweiten Hydroisomerisierungsreaktor und die Fraktionierkolonne überlagert, der in der oben geschilderten Weise dem 10 - 40-fachen des zur Oligomerisierung geführten Teilstroms entspricht. Der durch den zweiten Hydroisomerisierungsreaktor im Kreis gefahrene Teilstrom ist somit tendenziell höher. Sollte der vergrößerte Durchsatz durch den zweiten Hydroisomerisierungsreaktor durch die höher eingestellte LHSV nicht aufgefangen werden können, wird zweckmäßigerweise das Apparatevolumen dieses zweiten Hydroisomerisierungsreaktors vergrößert. Es ist selbstverständlich, daß die Fraktionierkolonne auf den höheren Durchsatz infolge des überlagerten Kreisstroms ausgelegt sein muß.

Im Reaktionsschritt d) des erfindungsgemäßen Verfahrens wird der zweite Teil des kondensierten Kopfprodukts der Oligomerisierung zugeführt. Die Oligomerisierung wird bei 30-140°C an einem sauren Katalysator durchgeführt. Als Katalysator für die Oligomerisierung werden heterogene anorganische oder organische Katalysatoren eingesetzt, beispielsweise saure Zeolithe, Kieselgele und saures Al₂O₃, saure Schicht- und Gerüstsilikate, mit Säuren belegte Trägermaterialien oder gelförmige oder makroporöse Kationenaustauscher in der H+-Form. Solche Katalysatoren sind dem Fachmann geläufig und werden von verschiedenen Herstellern auf dem Markt angeboten.

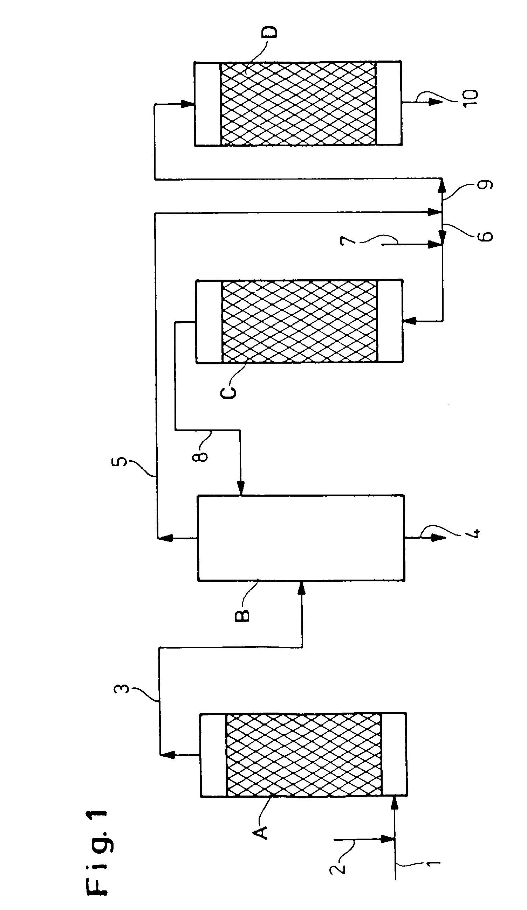

Die beigefügte Fig. 1 zeigt beispielhaft die Durchführung des erfindungsgemäßen Verfahrens: Einem ersten Hydroisomerisierungsreaktor A, der mit einem fest angeordneten Edelmetall-Hydrierkatalysator ausgestattet ist, wird über Leitung 1 flüssiges C₄-Raffinat I, gekennzeichnet durch einen Gehalt an zu oligomerisierendem i-Buten, zugeführt. Dem C₄-Raffinat I wird über Leitung 2 die erforderliche Menge H₂ zugemischt. A befindet sich im geflutetem Zustand und wird von unten nach oben befahren. Das A verlassende erste Hydroisomerisat wird über Leitung 3 der Destillationskolonne B zugeleitet, aus der als Sumpfprodukt 4 ein Gemisch abgezogen wird, das als wesentliche Bestandteile n-Butan und Buten-2 enthält. Das Kopfprodukt, das über Leitung 5 entnommen wird und neben hauptsächlich i-Buten und i-Butan nur noch wenig Buten-1 enthält, wird kondensiert und in zwei Anteile geteilt, die über die Leitungen 6 und 9 der weiteren Behandlung zugeführt werden. Der über 6 geführte Anteil wird über Leitung 7 mit H₂ vermischt und einem zweiten Hydroisomerisierungsreaktor C zugeführt, der wie A mit einem fest angeordneten Edelmetall-Hydrierkatalysator ausgestattet ist und ebenfalls von unten nach oben befahren wird. Das zweite Hydroisomerisat aus C wird über Leitung 8 dem oberen Teil von B zugeführt. Der über 9 geführte Anteil wird dem Oligomerisierungsreaktor D zugeführt, der mit einem sauren Katalysator, beispielsweise einem Kationenaustauscher in der H⁺-Form, ausgestattet ist. Der über 6 geführte Anteil ist in der Regel wesentlich größer als der über 9 geführte Anteil. Das über 10 abgezogene Oligomerisat wird in Fraktionen mit verschiedenen hohem Oligomerisierungsgrad aufgetrennt. Selbstverständliche und dem Fachmann bekannte Zusatzgeräte sind in Fig. 1 aus Gründen der Übersichtlichkeit nicht enthalten; dies betrifft Pumpen, Dosiereinrichtungen, Temperatur- und Druckmeßstellen, Vorwärmer, den Kondensator in Leitung 5, die Rückflußeinrichtung von 5 auf B, die Sumpfumlaufheizung am unteren Teil von B und die Auftrennung des Oligomerisats im Anschluß an D.

Das Oligomerisat des erfindungsgemäßen Verfahrens zeichnet sich vor allem durch zwei Vorteile aus: Zum einen zeigt sich im Bereich des Dimeren (DIB = Diisobutylen) ein Anstieg um mehr als 3 %-Punkte, typischerweise von 96 % auf über 99 % des gesamten Dimeren-Anteils, während der Anteil der Codimeren, also der Dimeren aus i-Buten und Buten-1, zurückgeht. Diese Codimeren sind, wie oben bereits erwähnt, weniger erwünscht als das DIB. Zum zweiten ergibt sich in überraschender Weise eine Verschiebung des gesamten Oligomerenspektrums vom DIB hin zu höheren Oligomerisierungsgraden, also hin zu C₁₂-, C₁₆- und C₂₀-Kohlenwasserstoffen.

### Beispiele

### Beispiel 1a (Hydroisomerisierung von Buten-1 zu Buten-2 in C₄-Raffinat I)

Ein Hydroisomerisierungsreaktor, der wie A in Fig. 1 mit den weiteren aus Fig. 1 ersichtlichen Apparaten verbunden war, wurde unter folgenden Bedingungen betrieben und ergab die in Tab. 1 und 2 aufgelisteten Ergebnisse: 200 mlKatalysator 0,3 Gew.-% Pd auf Al₂O₃ (Fa. Engelhard) wurden 24 h bei 200°C mit 200 l H₂/h aktiviert; 1150 g/h C₄-Raffinat I (LHSV = 10); Temp. 45°C (Reaktormitte); Druck 15 bar; 12 l/h H₂.

**Tab. 1:**

| C₄-Raffinat I-Einsatz und Hydroisomerisat in Abhängigkeit von der Laufzeit | | | | | | |
|---|---|---|---|---|---|---|
| Laufzeit (Tage) | Einsatz 0-7 Tage | Hydrogenisomerisat | | | | |
| | | 1 | 4 | 5 | 6 | 7 |
| i-Butan | 4,59 | 4,8 | 4,9 | 4,82 | 4,91 | 4,85 |
| n-Butan | 13,5 | 15,6 | 15,56 | 15,64 | 15,54 | 15,93 |
| i-Buten | 42,0 | 42,01 | 42,11 | 42,18 | 42,18 | 41,22 |
| Buten-1 | 23,6 | 4,65 | 4,38 | 4,13 | 3,98 | 3,81 |
| Buten-2 (cis, trans) | 16,0 | 32,41 | 32,23 | 32,56 | 32,66 | 33,56 |
| Butadien-1,3 | 0,22 | 0,00 | 0,00 | 0,00 | 0,00 | 0,00 |
| Höhersieder | 0,09 | 0,08 | 0,08 | 0,09 | 0,08 | 0,09 |

### Beispiel 1b (Destillation)

Eine Destillationskolonne von 8 m Länge und einem Durchmesser von 50 mm, gefüllt mit 6 mm Maschendrahtringen (theor. Bodenzahl: 120), die wie B mit den weiteren aus Fig. 1 ersichtlichen Apparaturen verbunden war, wurde kontinuierlich mit 1150 g/h Hydrogenisomerisat beschickt. Bei einem Druck von 12 bar, einer Kopftemperatur von 72°C und einer Sumpftemperatur von 86°C wurden 305 g/h Kopfprodukt, 8630 g/h Rücklaufmenge und 845 g/h Sumpfmenge eingestellt.

### Beispiel 1c (zweite Hydroisomerisation)

Die Rücklaufmenge aus Beispiel 1b wurde vor ihrer Rückführung in die Kolonne über einen Hydroisomerisierungsreaktor, der wie C mit den weiteren aus Fig. 1 ersichtlichen Apparaturen verbunden war, geleitet. Folgende Reaktionsbedingungen wurden eingestellt: 680 ml Katalysator 0,3 Gew.-% Pd auf Al₂O₃ (Fa. Engelhardt), wie unter Beispiel la aktiviert. 8630 g/h Rücklaufprodukt aus Beispiel 1b (LHSV = 22); Temperatur 48°C (Reaktormitte), Druck 12 bar, 10 l/h H₂. Das Kopfprodukt entsprach der Zusammensetzung des Einsatzes aus Beispiel 1d.

### Beispiel 1d (Oligomerisierung)

Ein Oligomerisierungsreaktor, der wie D in Fig. 1 mit den weiteren aus Fig. 1 ersichtlichen Apparaten verbunden war, wurde unter folgenden Bedingungen betrieben und ergab die in Tab. 3 aufgelisteten Ergebnisse: 167 mg Kationenaustauscher in der H⁺-Form als Katalysator; während 60 min. Durchsatz von 100 g erfindungsgemäßem Hydroisomerisat über Leitung 9 gemäß Fig. 1 der Zusammensetzung 9,88 % i-Butan, 1,00 % n-Butan, 86,69 % i-Buten, 0,59 % Buten-1, 1,62 % Buten-2 (cis, trans), 0,22 % Höhersieder bei drei verschiedenen Temperaturführungen (Versuche V1, V2, V3) bzw. zum Vergleich die gleiche Menge eines nicht erfindungsgemäßen Hydroisomerisats ohne Einsatz des Reaktors C der Zusammensetzung 4,47 % i-Butan, 13,92 % n-Butan, 42,55 % i-Buten, 22,52 % Buten-1, 16,06 % Buten-2 (cis, trans), 0,48 % Höhersieder (Vergleichsversuch Vergl.).

**Tab. 3:**

| Oligomerisate und daraus berechnete C₄-Raffinate II | | | | |
|---|---|---|---|---|
| | V 1 | V 2 | V 3 | Vergl. |
| Reakt. temp. (°C, Anfang) | 100-102 | 101-103 | 101-103 | 101-103 |
| Reakt. temp. (°C, max.) | 122,3 | 113,3 | 125,6 | 105 |
| C₄ (Rest-Raffinat I) | 13,11 % | 12,49 | 11,89 | 57,15 |
| C₈-Kohlenwasserstoffe | 31,82 | 36,53 | 33,15 | 27,83 |
| C₁₂-Kohlenwasserstoffe | 47,20 | 44,73 | 46,89 | 13,76 |
| C₁₆-Kohlenwasserstoffe | 7,33 | 5,89 | 7,42 | 1,26 |
| C₂₀-Kohlenwasserstoffe | 0,54 | 0,36 | 0,65 | - |

| Raffinat II | | | | |
|---|---|---|---|---|
| i-Butan | 67,89 | 69,42 | 70,65 | 8,52 |
| n-Butan | 7,78 | 7,93 | 8,16 | 25,73 |
| i-Buten | 9,38 | 6,81 | 5,30 | 1,08 |
| Buten-1 | 2,06 | 2,96 | 2,35 | 22,08 |
| Buten-2 (cis, trans) | 12,89 | 12,88 | 13,54 | 42,34 |
| Höhersieder | - | - | - | 0,25 |

Setzt man die Summe der erhaltenen C₈-C₂₀-Oligomere als 100 %-Wert; erhält man eine Verteilung wie in der folgenden Tab. 4:

**Tab. 4:**

| Oligomerenverteilung (%) | | | | |
|---|---|---|---|---|
| | V 1 | V 2 | V 3 | Vergl. |
| C₈ | 36,62 | 41,74 | 37,62 | 64,95 |
| C₁₂ | 54,32 | 51,12 | 53,22 | 32,11 |
| C₁₆ | 8,44 | 6,73 | 8,42 | 2,94 |
| C₂₀ | 0,62 | 0,41 | 0,74 | - |

Der erfindungsgemäße Anstieg der wertvolleren höheren C₁₂-C₂₀-Oligomere gegenüber dem C₈-Anteil wird deutlich. Auch innerhalb des Produktspektrums der C₈-Kohlenwasserstoffe gibt es eine vorteilhafte Verschiebung zugunsten der gewünschten Dimeren aus 2 Molekülen i-Buten ("Dimer") gegenüber sogenanntem "Codimer", das unter Beteiligung von Buten-1 entsteht (folgende Tab. 5).

**Tab. 5:**

| | V 1 | V 2 | V 3 | Vergl. |
|---|---|---|---|---|
| Dimer | 99,77 | 99,83 | 99,78 | 96,00 |
| Codimer | 0,23 | 0,17 | 0,22 | 4,00 |

## Patentansprüche

1. Verfahren zur Oligomerisierung von in einem C₄-Raffinat I enthaltenen i-Buten, dadurch gekennzeichnet, daß man
a) das C₄-Raffinat I einer ersten Hydroisomerisierung an einem Edelmetallkatalysator unterwirft, wobei bei 30-80°C, 5-30 bar, einer LHSV von 5-30 h⁻¹ und einer Menge von 3-20 NL gasförmigem H₂ pro Liter flüssigen C₄-Raffinats I, die über die zur Hydrierung hoch ungesättigter Komponenten im C₄-Raffinat benötigte Menge H₂ hinausgeht, gearbeitet wird,
b) das Hydroisomerisierungsprodukt von a) fraktioniert destilliert, wobei bei 5-25 bar und der sich zu diesem Druck einstellenden Temperatur gearbeitet wird und Buten-2 und n-Butan als Sumpfablauf und i-Buten und i-Butan als Kopfprodukt abgenommen werden,
c) das Kopfprodukt von b) kondensiert, in zwei Teile teilt und den ersten Teil einer zweiten Hydroisomerisierung an einem Edelmetallkatalysator unterwirft, wobei bei 30-80°C, 5-30 bar, einer LHSV von 5-30 h⁻¹ und einer Menge von 0,3-5 NL gasförmigem H₂ pro Liter Kondensat gearbeitet wird und die Hydroisomerisierungsbedingungen in a) und c) gleich oder verschieden sind, und das Hydroisomerisierungsprodukt von c) in das obere Drittel der Fraktionierungskolonne von b) zurückführt und
d) den zweiten Teil des kondensierten Kopfprodukts von b) bei 30-140°C an einem sauren Katalysator oligomerisiert.

2. Verfahren nach Anspruch 1, dadurch gekennzeichnet, daß die Aufteilung des kondensierten Kopfprodukts der Fraktionierkolonne so vorgenommen wird, daß auf 1 Volumteil des zur Oligomerisierung geführten Kondensats 10 bis 40 Volumteile, bevorzugt 20 bis 30 Volumteile, der zweiten Hydroisomerisierung zugeführt werden.

3. Verfahren nach Anspruch 1, dadurch gekennzeichnet, daß als Hydroisomerisierungskatalysator Ru, Rh, Pd, Ir, Pt oder mehrere von ihnen, bevorzugt Ru, Pd, Pt oder mehrere von ihnen, besonders bevorzugt Pd auf einem Träger eingesetzt wird.

4. Verfahren nach Anspruch 1, dadurch gekennzeichnet, daß zur Hydroisomerisierung der mit dem Katalysator ausgerüstete Reaktor von unten nach oben in gefluteter Form befahren wird.

5. Verfahren nach Anspruch 1, dadurch gekennzeichnet, daß die LHSV im Schritt a) auf 7-20 h⁻¹, bevorzugt auf 7-12 h⁻¹ eingestellt wird.

6. Verfahren nach Anspruch 1, dadurch gekennzeichnet, daß die LHSV im Schritt c) auf 10-25 h⁻¹, bevorzugt auf 15-25 h⁻¹ eingestellt wird.

## Claims

1. Process for oligomerizing i-butene present in a C₄ raffinate I, characterized in that
a) the C₄ raffinate I is subjected to a first hydroisomerization on a noble metal catalyst, with 30-80°C, 5-30 bar, an LHSV of 5-30 h⁻¹ and a rate of 3-20 L (S.T.P.) of gaseous H2 per litre of C₄ raffinate I which exceeds the amount of H₂ required to hydrogenate highly unsaturated components in the C₄ raffinate being employed,
b) the hydroisomerization product of a) is subjected to fractional distillation, 5-25 bar and the temperature established at this pressure being employed, and 2-butene and n-butane being taken off as bottom product and i-butene and i-butane as overhead product,
c) the overhead product of b) is condensed divided into two parts and the first part is subjected to a second hydroisomerization on a noble metal catalyst, with 30-80°C, 5-30 bar, an LHSV of 5-30 h⁻¹ and a rate of 0.3-5 L (S.T.P.) of gaseous H₂ per litre of condensate being employed and the hydroisomerization conditions in a) and c) being identical or different, and the hydroisomerization product of c) is recycled into the upper third of the fractionation column of b) and
d) the second part of the condensed overhead product of b) is oligomerized at 30-140°C on an acid catalyst.

2. Process according to Claim 1, characterized in that the division of the condensed overhead product of the fractionation column is performed in such a manner that, for 1 part by volume of the condensate fed to the oligomerization, 10 to 40 parts by volume, preferably 20 to 30 parts by volume, are fed to the second hydroisomerization.

3. Process according to Claim 1, characterized in that, as hydroisomerization catalyst, use is made of Ru, Rh, Pd, Ir, Pt or a plurality thereof, preferably Ru, Pd, Pt or a plurality thereof, particularly preferably Pd, on a support.

4. Process according to Claim 1, characterized in that, for the hydroisomerization, the flow passes through the reactor equipped with the catalyst from bottom to top in flooded form.

5. Process according to Claim 1, characterized in that the LHSV in step a) is set at 7-20 h⁻¹, preferably at 7-12 h⁻¹.

6. Process according to Claim 1, characterized in that the LHSV in step c) is set at 10-25 h⁻¹, preferably at 15-25 h⁻¹.

## Revendications

1. Procédé d'oligomérisation de l'i-butène contenu dans un produit raffiné en C4 I, caractérisé en ce que
a) on soumet le produit raffiné en C₄ I à une première hydroisomérisation sur un catalyseur de métal noble en opérant à 30-80°C, 5-30 bar, à une LHSV de 5-30 h⁻¹ et avec une quantité de 3-20 NL de H₂ gazeux par litre de produit raffiné en C₄ I, qui surpasse la quantité de H₂ nécessaire pour l'hydrogénation des composants hautement insaturés dans le produit raffiné en C₄,
b) on distille par distillation fractionnée le produit d'hydroisomérisation de a) en opérant à 5-25 bar et à la température qui s'établit à cette pression, et en retirant le but-2-ène et le n-butane sous forme d'écoulement de pied et l'i-butène et l'i-butane sous forme de produit de tête,
c) on condense le produit de tête de b), on le divise en deux parties et on soumet la première partie à une seconde hydroisomérisation sur un catalyseur de métal noble en opérant à 30-80°C, 5-30 bar, à une LHSV de 5-30 h⁻¹ et avec une quantité de 0,3-5 NL de H₂ gazeux par litre de condensat, les conditions d'hydroisomérisation en a) et c) étant identiques ou différentes, et on recycle le produit d'hydroisomérisation de c) dans le tiers supérieur de la colonne de fractionnement de b) et
d) on oligomérise la deuxième partie du produit de tête condensé de b) à 30-140°C sur un catalyseur acide.

2. Procédé selon la revendication 1, caractérisé en ce que la répartition du produit de tête condensé de la colonne de fractionnement est réalisée de telle manière que, pour 1 partie en volume de condensat envoyé à l'oligomérisation, 10 à 40 parties en volume, de préférence 20 à 30 parties en volume, sont envoyées à la seconde hydroisomérisation.

3. Procédé selon la revendication 1, caractérisé en ce que l'on utilise comme catalyseur d'hydroisomérisation Ru, Rh, Pd, Ir, Pt ou plusieurs d'entre eux, de préférence Ru, Pd, Pt ou plusieurs d'entre eux, de manière particulièrement préférée Pd sur un support.

4. Procédé selon la revendication 1, caractérisé en ce que, pour l'hydroisomérisation, le réacteur muni du catalyseur est parcouru de bas en haut sous forme noyée.

5. Procédé selon la revendication 1, caractérisé en ce que la LHSV dans l'étape a) est fixée à 7-20 h⁻¹, de préférence à 7-12 h⁻¹.

6. Procédé selon la revendication 1, caractérisé en ce que la LHSV dans l'étape c) est fixée à 10-25 h⁻¹, de préférence à 15-25 h⁻¹.
